# FASCICULE DE BREVET EUROPEEN

(11) **EP 2 854 912 B1**
(45) Date de publication et mention de la délivrance du brevet: **04.01.2017**
(21) Numéro de dépôt: 13731839.0
(22) Date de dépôt: 30.05.2013
(51) Int. Cl.: A61M 15/00

(54) **DISPOSITIF DE DISTRIBUTION DE PRODUIT FLUIDE DÉCLENCHÉ PAR L'INHALATION**
INHALATIONSBETÄTIGTE SPENDEVORRICHTUNG FÜR EIN FLÜSSIGES PRODUKT
DEVICE FOR DISPENSING A FLUID PRODUCT TRIGGERED BY INHALATION

(30) Priorité: 31.05.2012 FR 1255010
(43) Date de publication de la demande: 08.04.2015
(73) Titulaire: Aptar France SAS, 27110 Le Neubourg (FR)
(72) Inventeur: COLOMB, Arnaud, 78480 Verneuil Sur Seine (FR); KIRNIAK, Maxime, 76000 Rouen (FR)
(74) Mandataire: CAPRI
(86) Numéro de dépôt international: PCT/FR2013/051216
(87) Numéro de publication internationale: WO 2013/178951

(56) Documents cités:
- WO-A1-2006/079750
- WO-A2-2008/012458
- FR-A1- 2 909 645
- FR-A1- 2 918 353

## Description

La présente invention concerne un dispositif d'inhalation de produit fluide, et plus particulièrement un inhalateur de poudre sèche, déclenché par l'inhalation.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est d'abord chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Un autre problème qui peut se poser concerne l'assemblage de certaines pièces, notamment mobiles, qui doivent supporter des contraintes importantes en fonctionnement, et pour lesquels l'assemblage doit être particulièrement fiable pour éviter tout risque de dysfonctionnement. Avec la petite taille de certaines pièces, il peut être compliqué de garantir une telle fiabilité d'assemblage. Avec les inhalateurs qui sont chargés, par exemple lors de leur ouverture, puis déclenchés par l'inhalation, il est important d'éviter ou de limiter les risques de déclenchements intempestifs après chargement et avant inhalation, sans imposer un seuil d'inhalation trop élevé pour réaliser le déclenchement, qui pourrait être difficilement accessible à des personnes affaiblies. Un tel inhalateur de poudre sèche déclenché par l'inhalation est connu, par exemple, du document WO 2008/012458 A2.

La présente invention a pour but de fournir un dispositif d'inhalation de produit fluide, en particulier un inhalateur de poudre sèche qui ne reproduit pas les inconvénients susmentionnés.

En particulier, la présente invention a pour but de fournir un tel dispositif qui soit simple et peu coûteux à fabriquer et à assembler, fiable à l'assemblage et en utilisation, garantissant une précision et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a donc pour objet un dispositif d'inhalation de produit fluide déclenché par l'inhalation, comportant un corps principal, au moins un élément de capot monté pivotant sur ledit corps principal entre une position fermée et une position ouverte, ledit dispositif comportant au moins un réservoir individuel contenant une dose unique de produit fluide, tel que de la poudre, des moyens d'ouverture étant prévus pour ouvrir un réservoir individuel à chaque actionnement du dispositif, ledit dispositif comportant des moyens de support mobiles adaptés à déplacer un réservoir individuel contre lesdits moyens d'ouverture à chaque actionnement, lesdits moyens de support mobiles étant déplaçables entre une position de non-distribution et une position de distribution, lesdits moyens de support mobiles étant sollicités vers leur position de distribution par des moyens élastiques, tel qu'un ressort, et étant retenus en position de non-distribution par des moyens de blocage qui sont libérés par l'inhalation de l'utilisateur, ledit dispositif comportant un organe d'armement coopérant avec lesdits moyens élastiques et avec une surface de came, formée sur lesdits moyens de support mobiles, ladite surface de came comportant au moins trois parties de came avec des pentes différentes, dont au moins une partie de chargement pour charger lesdits moyens élastiques, au moins une partie d'extrémité pour caler ledit organe d'armement dans ladite position ouverte et/ou dans ladite position fermée dudit au moins un élément de capot, et au moins une partie de sécurité pour relier au moins une partie de chargement à au moins une partie d'extrémité, ledit organe d'armement étant déplaçable sur ladite partie de sécurité sans compression supplémentaire et sans décompression desdits moyens élastiques, ledit dispositif étant dans une position stable si ledit organe d'armement est arrêté sur ladite partie de sécurité.

Avantageusement, ladite au moins une partie de chargement est de forme plane et rectiligne.

Avantageusement, ladite au moins une partie de sécurité est en forme d'arc de cercle, de sorte qu'aucune force latérale n'est exercée sur ledit organe d'armement lorsqu'il coopère avec ladite partie de sécurité.

Avantageusement, ladite au moins une partie d'extrémité est plane ou en forme de V.

Avantageusement, ladite surface de came comporte, en partant de la position fermée, une partie de chargement, une partie de sécurité et une partie d'extrémité.

Avantageusement, le dispositif comporte un système de déclenchement par l'inhalation qui comporte une chambre d'air déformable coopérant avec un embout d'inhalation, et un élément déclencheur coopérant avec ladite chambre d'air, de sorte que lors d'une inhalation à travers ledit embout d'inhalation, ladite chambre d'air est déformée et ledit élément déclencheur actionne lesdits moyens d'ouverture, de sorte que lors d'une inhalation à travers l'embout d'inhalation, un réservoir est ouvert par lesdits moyens d'ouverture.

Avantageusement, lesdits moyens d'ouverture comportent un élément de perçage fixe par rapport audit corps principal, adapté à découper une paroi de fermeture du réservoir de telle sorte que la ou les partie(s) découpée(s) n'obstrue(nt) pas la ou les ouverture(s) formée(s).

Ces caractéristiques et avantages et d'autres de la présente invention apparaîtront plus clairement au cours de la description détaillée suivante, faite en référence aux dessins joints, donnés à titre d'exemples non limitatifs, sur lesquels
- les figures 1 et 2 sont des vues schématiques en section transversale d'un dispositif d'inhalation selon un premier mode de réalisation de l'invention, respectivement en positions fermée et ouverte des capots,
- la figure 3 est une vue schématique de détail de l'organe d'armement dans une première position intermédiaire, entre les positions fermée et ouverte des capots,
- la figure 4 est une vue similaire à celle de la figure 3, dans une seconde position intermédiaire,
- la figure 5 est une vue similaire à celles des figures 3 et 4, dans la position ouverte des capots,
- les figures 6 et 7 sont des vues similaires à celles des figures 1 et 2, représentant un second mode de réalisation de l'invention,
- les figures 8 à 10 sont des vues similaires à celles des figures 3 à 5, montrant le dispositif des figures 6 et 7 respectivement dans les positions fermée, intermédiaire et ouverte des capots, et
- les figures 11 et 12 sont des vues similaires à celles des figures 1 et 2, représentant un troisième mode de réalisation de l'invention.

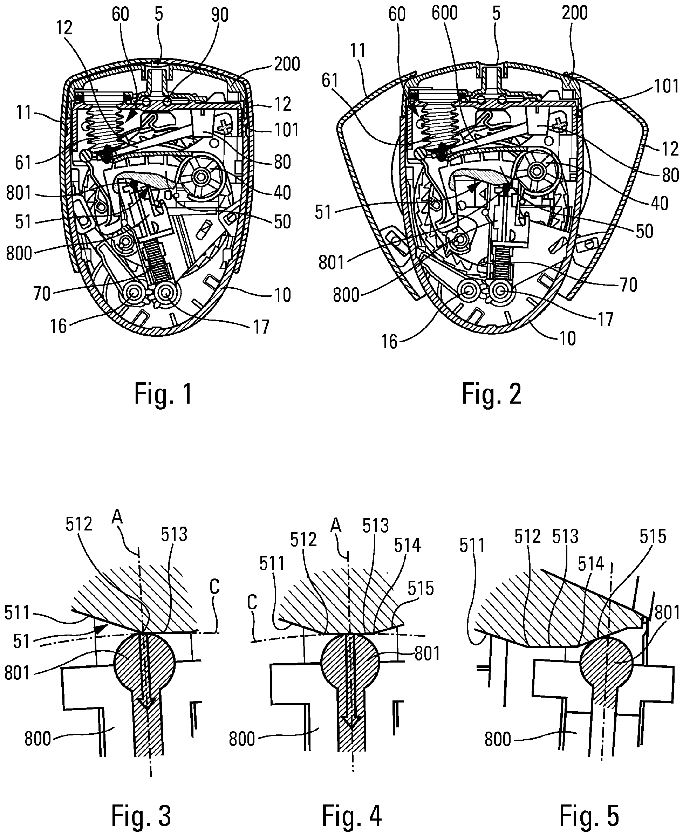

Sur les figures 1 et 2, il est représenté un exemple avantageux d'un inhalateur de poudre sèche. Cet inhalateur comporte un corps principal 10 sur lequel peuvent être montées coulissantes deux parties 11, 12 formant capot, adaptées à être ouvertes pour ouvrir et charger le dispositif. Le corps principal 10 peut être de forme environ arrondie comme représenté sur les figures, mais il pourrait avoir toute autre forme appropriée. Un corps supérieur 101 est assemblé au corps principal 10, et un embout buccal 200 est assemblé sur ledit corps supérieur 101. Cet embout buccal 200 définit un orifice de distribution 5 à travers lequel l'utilisateur va inhaler lors de l'actionnement du dispositif. Cet orifice de distribution 5 est typiquement disposé environ au centre de l'embout buccal 200. Les capots 11, 12 peuvent s'ouvrir par pivotement autour d'un axe de rotation commun, ou autour de deux axes parallèles en étant engrené l'un avec l'autre. Tout autre moyen d'ouverture du dispositif est envisageable. En variante, le dispositif pourrait ne comporter qu'un seul capot au lieu de deux.

A l'intérieur du corps principal 10, il est prévu une bande de réservoirs individuels (non représentée dans des buts de clarté), également appelés blisters, réalisée sous forme d'une bande allongée sur laquelle les blisters sont disposés les uns derrière les autres, de manière connue. Cette bande de blisters est avantageusement constituée d'une couche ou paroi de base formant les cavités recevant les doses de poudre, et d'une couche ou paroi de fermeture qui obture de manière étanche chacun desdits blisters. Avant la première utilisation, la bande de blisters peut être enroulée à l'intérieur du corps principal 10, de préférence dans une partie de stockage, et des premiers moyens de déplacement de bande 40, notamment rotatifs, sont prévus pour progressivement dérouler et faire avancer cette bande de blisters.

Des seconds moyens de déplacement 50, notamment pivotants sur le corps principal 10, sont prévus pour amener un blister respectif dans une position de distribution à chaque actionnement du dispositif. Ces seconds moyens de déplacement sont avantageusement pivotants entre une position de non-distribution et une position de distribution, dans laquelle un blister coopère avec lesdits moyens d'ouverture.

La partie de bande comportant les blisters vides est avantageusement adaptée à s'enrouler dans un autre endroit dudit corps 10, de préférence une partie de réception, comme cela sera décrit plus en détail ci-après.

L'inhalateur comporte des moyens d'ouverture de blister 80 (seulement représentés très schématiquement dans des buts de clarté) comportant de préférence une aiguille de perçage et/ou de coupage de la couche de fermeture des blisters. De préférence, les moyens d'ouverture comportent un élément de perçage 80, fixe par rapport aux corps principal 10 et supérieur 101, et contre lequel un blister respectif est déplacé à chaque actionnement par les seconds moyens de déplacement. Le blister est alors percé par ledit élément de perçage, qui pénètre dans ledit blister pour expulser la poudre au moyen du flux d'inhalation de l'utilisateur. Avantageusement, l'élément de perçage est adapté à découper une paroi de fermeture du réservoir de telle sorte que la ou les partie(s) découpée(s) n'obstrue(nt) pas la ou les ouverture(s) formée(s). Les documents WO 2006/079750 et WO 2009/007640 décrivent de tels moyens d'ouverture de blister.

Les premiers moyens de déplacement 40 sont adaptés à faire avancer la bande de blisters après chaque inhalation de l'utilisateur. Les seconds moyens de déplacement 50 sont adaptés à déplacer le blister à vider contre lesdits moyens d'ouverture lors de l'actionnement, avant chaque inhalation. Ces seconds moyens de déplacement sont sollicités par un élément élastique 70, tel qu'un ressort ou tout autre élément élastique équivalent, ledit élément élastique pouvant être pré-chargé lors de l'ouverture du dispositif.

De préférence, les premiers moyens de déplacement 40 sont formés par une roue d'indexage qui reçoit et guide la bande de blisters. La suite de la description sera donc faite en référence à une telle roue d'indexage 40. Une rotation de cette roue d'indexage 40 fait avancer la bande de blisters. Avant chaque inhalation, un blister plein est toujours en position face aux moyens d'ouverture 80. Les seconds moyens de déplacement 50 peuvent comporter un organe pivotant autour d'un axe de rotation, ladite roue d'indexage 40 étant avantageusement montée rotative sur ledit organe pivotant.

Un cycle d'actionnement du dispositif peut être le suivant. Lors de l'ouverture du dispositif, les deux parties latérales 11, 12 formant capot sont écartées l'une de l'autre en pivotant sur le corps pour ouvrir le dispositif, et ainsi charger le dispositif. Dans cette position la roue d'indexage 40 ne peut pas se déplacer vers l'élément de perçage 80 car les seconds moyens de déplacement 50 sont retenus par des moyens de blocage appropriés (non représentés dans des buts de clarté). Les documents WO 2009/077700 et WO 2009/136098 décrivent de tels moyens de blocage.

Lors de l'inhalation par l'utilisateur à travers l'embout buccal, ces moyens de blocage sont débloqués, ce qui provoque alors le déplacement de ladite roue d'indexage 40 en direction de l'aiguille, et donc l'ouverture d'un blister.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture, on prévoit un système de déclenchement par l'inhalation 60 qui comporte avantageusement une chambre d'air 61 déformable sous l'effet de l'inhalation, cette chambre d'air étant adaptée à libérer les moyens de blocage. La chambre d'air 61 peut avantageusement être réalisée en forme de soufflet. L'inhalation de l'utilisateur provoque la déformation de ladite chambre d'air déformable, libérant ainsi lesdits moyens de blocage et permettant donc le déplacement des seconds moyens de déplacement, et donc d'un blister respectif, vers sa position d'ouverture. Le blister n'est donc ouvert qu'au moment de l'inhalation, de sorte qu'il est simultanément vidé. Il n'y a donc aucun risque de perte de dose entre l'ouverture du blister et son vidage.

L'inhalateur comporte en outre une chambre de dispersion 90 qui est destinée à recevoir la dose de poudre après ouverture d'un blister respectif. Avantageusement, cette chambre de dispersion est pourvue d'au moins une bille, de préférence plusieurs, qui se déplace(nt) à l'intérieur de ladite chambre 90 pendant l'inhalation, notamment pour améliorer la distribution du mélange air et poudre après ouverture d'un blister, afin d'augmenter l'efficacité du dispositif.

Il peut être avantageux que les moyens d'ouverture, en particulier l'élément de perçage, soient directement reliés à ladite chambre de dispersion, par exemple via un canal menant à ladite chambre 90.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les blisters sont formés sur une bande allongée souple, qui, au début, est principalement stockée sous forme de bobine dans un logement de stockage à l'intérieur du corps principal 10 du dispositif. Avantageusement, cette bande de blisters enroulée est maintenue par des parois internes dudit logement de stockage sans que son extrémité arrière (dans le sens d'avancement de la bande de blisters) ne soit fixée par rapport audit corps principal 10, ce qui permet un assemblage plus aisé de cette bobine de bande de blisters à l'intérieur du dispositif. La bande de blisters est déplacée au moyen de la roue d'indexage 40 qui présente avantageusement au moins un, de préférence plusieurs évidements, dont la forme correspond à celle des blisters. Ainsi, lorsque cette roue d'indexage 40 tourne, elle fait avancer la bande de blisters. Bien entendu en variante ou de manière additionnelle, on pourrait utiliser d'autres moyens d'avancée de bande de blisters, par exemple en prévoyant un profil sur les bords longitudinaux latéraux de la bande de blisters, ledit profil étant adapté à coopérer avec des moyens d'entraînement appropriés. De même, des trous ménagés le long des bords latéraux de la bande de blisters pourraient également être utilisés pour faire avancer la bande de blisters au moyen de roues dentées coopérant avec lesdits trous.

Après ouverture d'un ou plusieurs blister(s), la partie de bande de blisters avec les blisters vidés doit pouvoir être stockée de manière aisée et non encombrante dans le dispositif, en évitant tout risque de blocage. Avantageusement, cette bande de blisters usée s'enroule automatiquement sur elle-même pour à nouveau former une bobine.

Selon encore un autre aspect de l'inhalateur, un dispositif de comptage ou d'indication de doses (non représenté dans des buts de clarté) peut également être prévu. Ce dispositif peut comporter des chiffres ou symboles inscrits directement sur la bande de blister et visibles à travers une fenêtre appropriée dans le corps principal 10 du dispositif. En variante, on pourrait envisager d'utiliser un compteur avec un ou plusieurs disque(s) ou anneau(x) rotatif(s) comportant des numéros ou symboles. Les documents WO 2008/012458 et WO 2011/154659 décrivent de tels compteurs.

Afin d'éviter de compter des doses qui ne sont pas distribuées, par exemple en cas d'erreur de manipulation, ou d'une manipulation incomplète du dispositif, il est souhaitable que le compteur ou indicateur ne soit actionné qu'une fois que l'utilisateur a inhalé, puisque c'est cette inhalation qui conditionne l'ouverture et la distribution de la dose de chaque blister. Avantageusement, l'actionnement du compteur se fait donc après inhalation, lorsque l'utilisateur referme le dispositif.

L'élément de capot mobile 12 est solidaire d'un organe d'armement 800 qui peut coulisser dans un logement approprié. Cet organe d'armement 800 est avantageusement pivotant par rapport audit corps 10 ensemble avec l'élément de capot 12. Cet organe d'armement 800 peut se déplacer contre le ressort 70, avantageusement un ressort à boudins, disposé dans ledit logement. L'organe d'armement 800 est donc connecté d'un côté audit ressort 70 et de l'autre côté, il coopère avec les seconds moyens de déplacement, en particulier avec un organe 50 pivotant sur le corps 10, et sur lequel est fixée de manière rotative la roue d'indexage 40.

Lorsque l'élément de capot mobile 12 est ouvert, l'organe d'armement 800 est déplacé dans son logement en comprimant le ressort 70. L'organe pivotant 50 des seconds moyens de déplacement est lui empêché de se déplacer par les moyens de blocage susmentionnés, qui ne seront libérés qu'au moment de l'inhalation. Ainsi, en l'absence d'inhalation dans la position ouverte, la fermeture des éléments de capot 11, 12 provoquerait simplement le retour à la position de repos pour l'organe d'armement 800, et la décompression du ressort 70.

Ainsi, lorsque l'utilisateur ouvre l'inhalateur il charge le système. S'il n'inhale pas et qu'il referme l'inhalateur, celui-ci reprend simplement sa position de départ sans déplacer la bande de blisters ni les moyens de blocage. Il n'y a donc aucun risque qu'un blister (et donc une dose de produit actif) soit perdu par un actionnement malencontreux ou incomplet dans lequel l'utilisateur n'exercerait pas d'inhalation entre l'ouverture et la fermeture. L'ouverture du blister, son vidage, la distribution de la poudre dans les poumons de l'utilisateur, le déplacement de la bande de blisters pour amener un nouveau blister plein en face des moyens d'ouverture ainsi que le comptage de la dose ne sont donc possibles que si l'utilisateur inhale.

Les moyens de blocage, qui bloquent les seconds moyens de déplacement et notamment l'organe pivotant qui coopère avec l'organe d'armement, sont connectés à la chambre d'air déformable 61 sensible à l'inhalation de l'utilisateur par l'intermédiaire d'un élément déclencheur 600, de sorte que lors de l'inhalation de l'utilisateur, ladite chambre d'air déformable se déforme, faisant pivoter l'élément déclencheur 600, et libérant ainsi lesdits moyens de blocage. Ceci permet le déplacement desdits seconds moyens de déplacement vers leur position de distribution sous l'effet de la force exercée par le ressort comprimé 70 sur l'organe d'armement 800 qui pousse sur l'organe pivotant 50. Ce déplacement provoque l'ouverture d'un blister plein et la distribution d'une dose.

Une surface de came 51 est formée sur lesdits moyens de support mobiles 50, sur laquelle glisse l'organe d'armement 800. L'organe d'armement 800 est donc adaptée à comprimer le ressort 70 lorsque l'élément de capot 12 est ouvert, et à décomprimer ledit ressort 70 lorsque ledit élément de capot 12 est refermé.

Avantageusement, l'organe d'armement 800 comporte, dans sa partie en contact avec la surface de came 51, une partie arrondie 801 pour favoriser le glissement de l'organe d'armement 800 sur ladite surface de came 51.

Les moyens de support mobiles sont dans ce mode de réalisation réalisés sous la forme d'un organe 50 monté pivotant sur le corps 10 autour d'un axe de pivotement. La surface de came précitée 51 étant formée sur ledit organe pivotant 50, lorsque le ressort 70 est chargé lors de l'ouverture de l'élément de capot mobile 12, ledit organe pivotant 50 est sollicité vers sa position de distribution par ledit organe d'armement 800 et le ressort 70 comprimé.

Après l'inhalation, c'est-à-dire en position de distribution, les moyens de blocage ont été libérés et les moyens de support mobiles 50 ont été déplacés vers le haut par le ressort comprimé 70.

Avantageusement, les deux éléments de capot mobiles 11, 12 sont engrenés l'un dans l'autre via des engrenages appropriés pour garantir une ouverture et une fermeture symétrique desdits deux éléments de capot mobiles. Cet engrenage peut se faire à proximité de leur axe de pivotement 16, 17.

Selon l'invention, la surface de came 51 comporte au moins trois parties de came avec des pentes différentes. Au moins une partie de chargement est prévue pour déplacer l'organe d'armement 800 pour charger le ressort 70. Au moins une partie d'extrémité est prévue pour bloquer l'organe d'armement 800 en position ouverte et/ou fermée des capots 11,12. Au moins une partie de sécurité est prévue, disposée directement avant et/ou après une partie d'extrémité, pour assurer un fonctionnement fiable. Dans cette partie de sécurité, l'organe d'armement 800 se déplace sans compression et/ou décompression supplémentaire du ressort 70.

Les figures 1 à 5 représentent un premier mode de réalisation. Dans cette variante, la surface de came comporte une partie de chargement 511 sensiblement rectiligne. Une partie de sécurité 513 se connecte à ladite partie de chargement au niveau d'un premier sommet 512. Cette partie de sécurité 513 a de préférence une forme en arc de cercle, et se connecte à une partie d'extrémité 515 au niveau d'un second sommet 514.

En partant de la position fermée de la figure 1, lorsque l'utilisateur ouvre les capots 11, 12, l'organe d'armement 800 va d'abord glisser sur la partie de chargement 511. Ceci va faire coulisser l'organe d'armement 800 dans son logement pour charger le ressort 70.

Lorsque l'organe d'armement arrive au premier sommet 512, comme représenté sur la figure 3, le ressort est chargé, c'est-à-dire comprimé. Cette position est atteinte dans une première position intermédiaire, dans laquelle les capots 11, 12 ne sont pas complètement ouverts.

La poursuite de l'ouverture des capots amène l'organe d'armement 800 à glisser sur la partie de sécurité 513. Cette partie de sécurité pourrait être plane, mais elle est de préférence en arc de cercle C, avec le rayon de cet arc de cercle correspondant à celui du mouvement de pivotement de l'organe d'armement 800 lors de l'ouverture des capots. De ce fait, aucune force latérale n'est exercée sur l'organe d'armement 800 pendant qu'il se déplace sur la partie de sécurité 513. Seule la réaction axiale à la force du ressort 70 chargée s'applique sur l'organe d'armement 800 le long de son axe longitudinal A, comme représenté par la flèche sur la figure 4. Si le mouvement d'ouverture ou de fermeture des capots devait s'arrêter sur cette partie de sécurité 513, par exemple dans la seconde position intermédiaire de la figure 4, le dispositif serait dans une position stable. De même, du fait de cette forme en arc de cercle, il n'y a plus de coulissement axial de l'organe d'armement 800 lorsqu'il se déplace dans cette partie de sécurité. Le ressort 70 est donc complètement chargé à la fin de la partie de chargement 511, au niveau du premier sommet 512. La fin de l'ouverture, sur ladite partie de sécurité 513 et jusqu'à la partie d'extrémité 515 est donc aisé, puisqu'elle n'implique plus de compression supplémentaire ni décompression du ressort 70.

Lorsque les capots sont complètement ouverts, l'organe d'armement vient coopérer avec la partie d'extrémité 515. Cette partie d'extrémité peut être formée par une partie plane, comme représenté sur la figure 5, ou avoir une forme en V, pour caler, au moins légèrement, l'organe d'armement 800, et donc les capots 11, 12, en position ouverte des capots.

La position ouverte est bien calée, mais néanmoins, lors de l'actionnement, des forces importantes sont libérées, ce qui pourrait dans certain cas provoquer la sortie intempestive et non souhaitée de l'organe d'armement 800 hors de la partie d'extrémité 515. Dans un tel cas, la partie de sécurité 513 agit pour maintenir le dispositif dans une position stable, permettant un actionnement fiable de l'inhalateur. Ceci ne serait pas le cas si la partie d'extrémité était directement connectée à la partie de chargement 511.

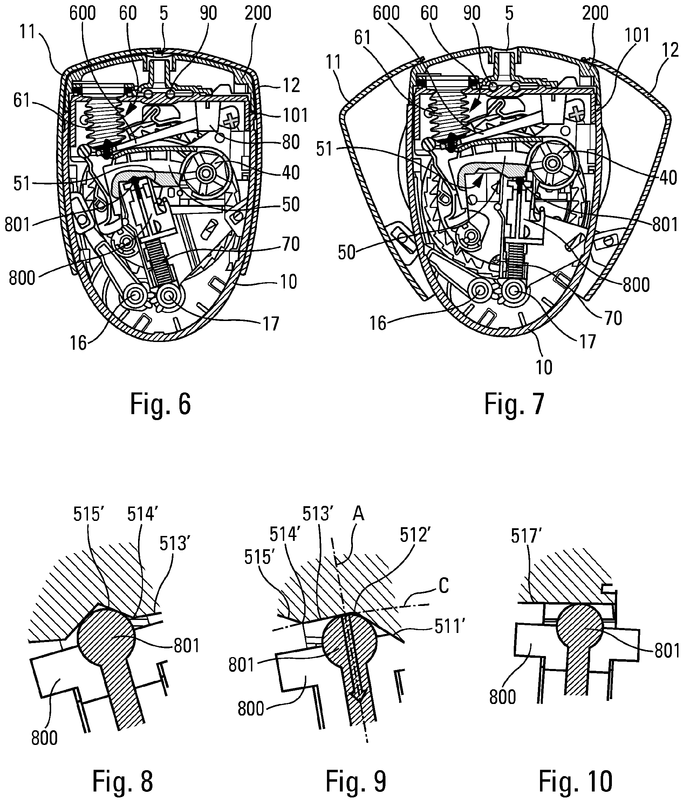

Les figures 6 à 10 illustrent un second mode de réalisation, qui est sensiblement inversé par rapport au premier mode de réalisation des figures 1 à 5. Ici, la partie d'extrémité 515' est au début de la surface de came 51, dans la séquence d'ouverture des capots, suivie, après un sommet 514', par la partie de sécurité 513', elle-même reliée à la partie de chargement 511' via un autre sommet 512'. La partie de chargement 511' se connecte alors dans une partie finale 517', visible sur la figure 10, qui peut être plane ou en arc de cercle. Dans cet exemple, la partie d'extrémité 515' est en forme de V, comme visible sur la figure 8, mais elle pourrait aussi être de forme environ plane.

Le fonctionnement est donc similaire mais inversé par rapport au premier mode de réalisation. C'est dans la position fermée des capots que l'organe d'armement 800 est calé dans la partie d'extrémité 515', ce qui permet de garantir une bonne position fermée des capots.

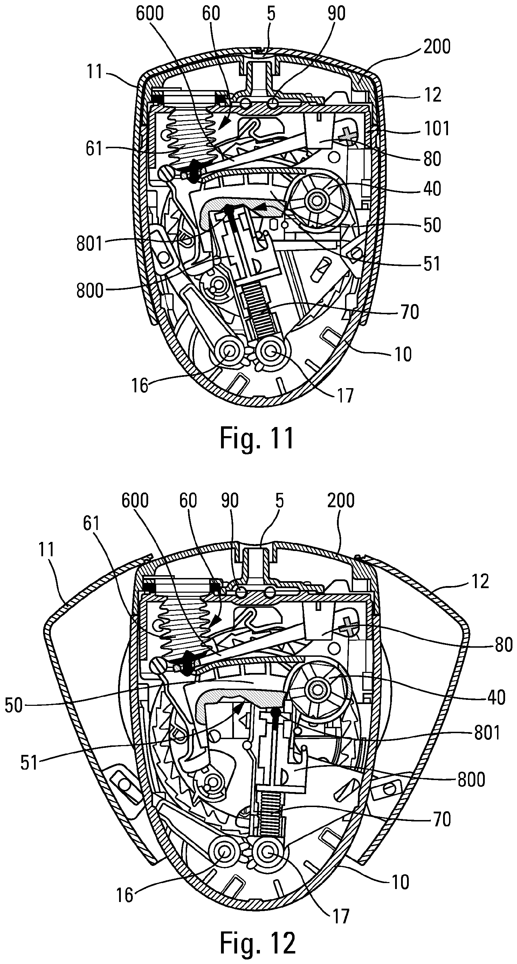

Les figures 11 et 12 illustrent un troisième mode de réalisation, dans lequel sont combinés les premier et second modes de réalisations des figures 1 à 5 et 6 à 10.

Dans cette variante, les deux positions ouverte et fermée des capots sont bien définies par des parties d'extrémités, et la partie de chargement sensiblement centrale est reliée à chaque partie d'extrémité par une partie de sécurité respective. Les avantages des premier et second modes de réalisation se combinent dans ce troisième mode de réalisation.

Un inhalateur de poudre sèche tel que décrit précédemment procure notamment les fonctions suivantes :
▪ une pluralité de doses individuelles de poudre stockées dans des blisters individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération pré-chargé ;
▪ des moyens d'entraînement de forme appropriée en prises avec les blisters pour réaliser le déplacement de la bande de blisters après chaque inhalation, et amener un nouveau blister plein dans une position dans laquelle il est destiné à être ouvert par les moyens d'ouverture appropriés ;
▪ des moyens pour éviter une perte de dose en cas d'ouverture de l'inhalateur mais en l'absence d'inhalation ;
▪ un indicateur de doses adapté à compter les doses seulement en cas d'inhalation.

D'autres fonctions sont également fournies par le dispositif de l'invention tel que cela a été décrit précédemment.

Il est à noter que les différentes fonctions, même si elles ont été représentées comme prévues simultanément sur l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les blisters individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Dispositif d'inhalation de produit fluide déclenché par l'inhalation, comportant un corps principal (10), au moins un élément de capot (11, 12) monté pivotant sur ledit corps principal (10) entre une position fermée et une position ouverte, ledit dispositif comportant au moins un réservoir individuel contenant une dose unique de produit fluide, tel que de la poudre, des moyens d'ouverture (80) étant prévus pour ouvrir un réservoir individuel à chaque actionnement du dispositif, ledit dispositif comportant des moyens de support mobiles (50) adaptés à déplacer un réservoir individuel contre lesdits moyens d'ouverture (80) à chaque actionnement, lesdits moyens de support mobiles (50) étant déplaçables entre une position de non-distribution et une position de distribution, lesdits moyens de support mobiles (50) étant sollicités vers leur position de distribution par des moyens élastiques (70), tel qu'un ressort, et étant retenus en position de non-distribution par des moyens de blocage qui sont libérés par l'inhalation de l'utilisateur, ledit dispositif comportant un organe d'armement (800) coopérant avec lesdits moyens élastiques (70) et avec une surface de came (51), formée sur lesdits moyens de support mobiles (50), **caractérisé en ce que** ladite surface de came (51) comporte au moins trois parties de came avec des pentes différentes, dont au moins une partie de chargement (511 ; 511') pour charger lesdits moyens élastiques (70), au moins une partie d'extrémité (515 ; 515') pour caler ledit organe d'armement (800) dans ladite position ouverte et/ou dans ladite position fermée dudit au moins un élément de capot (11, 12), et au moins une partie de sécurité (513 ; 513') pour relier au moins une partie de chargement (511 ; 511') à au moins une partie d'extrémité (515 ; 515'), ledit organe d'armement étant déplaçable sur ladite partie de sécurité (513 ; 513') sans compression supplémentaire et sans décompression desdits moyens élastiques (70), ledit dispositif étant dans une position stable si ledit organe d'armement (800) est arrêté sur ladite partie de sécurité (513 ; 513').

2. Dispositif selon la revendication 1, dans lequel ladite au moins une partie de chargement (511 ; 511') est de forme plane et rectiligne.

3. Dispositif selon la revendication 1 ou 2, dans lequel ladite au moins une partie de sécurité (513 ; 513') est en forme d'arc de cercle, de sorte qu'aucune force latérale n'est exercée sur ledit organe d'armement (800) lorsqu'il coopère avec ladite partie de sécurité.

4. Dispositif selon l'une quelconque des revendications précédentes, dans lequel ladite au moins une partie d'extrémité (515 ; 515') est plane ou en forme de V.

5. Dispositif selon l'une quelconque des revendications précédentes, dans lequel ladite surface de came (51) comporte, en partant de la position fermée, une partie de chargement (511), une partie de sécurité (513) et une partie d'extrémité (515).

6. Dispositif selon l'une quelconque des revendications précédentes, comportant un système de déclenchement par l'inhalation (60) qui comporte une chambre d'air déformable (61) coopérant avec un embout d'inhalation (200), et un élément déclencheur (600) coopérant avec ladite chambre d'air (61), de sorte que lors d'une inhalation à travers ledit embout d'inhalation (200), ladite chambre d'air (61) est déformée et ledit élément déclencheur (600) actionne lesdits moyens d'ouverture (80), de sorte que lors d'une inhalation à travers l'embout d'inhalation (200), un réservoir est ouvert par lesdits moyens d'ouverture.

7. Dispositif selon l'une quelconque des revendications précédentes, dans lequel lesdits moyens d'ouverture (80) comportent un élément de perçage fixe par rapport audit corps principal (10), adapté à découper une paroi de fermeture du réservoir de telle sorte que la ou les partie(s) découpée(s) n'obstrue(nt) pas la ou les ouverture(s) formée(s).

## Patentansprüche

1. Inhalationsvorrichtung für ein fluides Produkt, die durch Inhalation ausgelöst wird, aufweisend einen Hauptkörper (10), mindestens ein Kappenelement (11, 12), das auf dem Hauptkörper (10) zwischen einer geschlossenen Position und einer geöffneten Position schwenkbar montiert ist, wobei die Vorrichtung mindestens einen einzelnen Behälter aufweist, der eine einzige Dosis von fluidem Produkt, wie ein Pulver, enthält, wobei Öffnungsmittel (80) vorgesehen sind, um bei jeder Betätigung der Vorrichtung einen einzelnen Behälter zu öffnen, wobei die Vorrichtung bewegliche Trägermittel (50) aufweist, die dazu geeignet sind, bei jeder Betätigung einen einzelnen Behälter gegen die Öffnungsmittel (80) zu verschieben, wobei die beweglichen Trägermittel (50) zwischen einer Nichtausgabeposition und einer Ausgabeposition verschiebbar sind, wobei die beweglichen Trägermittel (50) durch elastische Mittel (70), wie eine Feder, in ihre Ausgabeposition belastet und durch Sperrmittel in der Nichtausgabeposition gehalten werden, die durch Inhalation durch den Benutzer freigegeben werden, wobei die Vorrichtung eine Spanneinrichtung (800) aufweist, die mit den elastischen Mitteln (70) und mit einer Nockenfläche (51) zusammenwirkt, die auf den beweglichen Trägermitteln (50) gebildet ist, **dadurch gekennzeichnet, dass** die Nockenfläche (51) mindestens drei Nockenteile mit verschiedenen Steigungen aufweist, davon mindestens einen Belastungsteil (511; 511'), um die elastischen Mittel (70) zu belasten, mindestens einen Endteil (515 ; 515'), um die Spanneinrichtung (800) in der geöffneten Position und/oder in der geschlossenen Position des mindestens einen Kappenelements (11, 12) festzusetzen, und mindestens einen Sicherheitsteil (513; 513'), um mindestens einen Belastungsteil (511; 511') mit mindestens einem Endteil (515; 515') zu verbinden, wobei die Spanneinrichtung auf dem Sicherheitsteil (513; 513') ohne zusätzliches Zusammendrücken und ohne Druckminderung der elastischen Mittel (70) verschiebbar ist, wobei die Vorrichtung in einer stabilen Position ist, wenn die Spanneinrichtung (800) auf dem Sicherheitsteil (513; 513') festgestellt ist.

2. Vorrichtung nach Anspruch 1, wobei der mindestens eine Belastungsteil (511; 511') von einer ebenen und geradlinigen Form ist.

3. Vorrichtung nach Anspruch 1 oder 2, wobei der mindestens eine Sicherheitsteil (513; 513') kreisbogenförmig ist, so dass keine seitliche Kraft auf die Spanneinrichtung (800) ausgeübt wird, wenn sie mit dem Sicherheitsteil zusammenwirkt.

4. Vorrichtung nach einem der vorhergehenden Ansprüche, wobei der mindestens eine Endteil (515; 515') eben oder V-förmig ist.

5. Vorrichtung nach einem der vorhergehenden Ansprüche, wobei die Nockenfläche (51), wenn sie die geschlossene Position verlässt, einen Belastungsteil (511), einen Sicherheitsteil (513) und einen Endteil (515) aufweist.

6. Vorrichtung nach einem der vorhergehenden Ansprüche, aufweisend ein Auslösesystem durch Inhalation (60), welches eine verformbare Luftkammer (61), die mit einem Inhalationsansatz (200) zusammenwirkt, und ein Auslöseelement (600), das mit der Luftkammer (61) zusammenwirkt, aufweist, so dass die Luftkammer (61) bei einer Inhalation durch den Inhalationsansatz (200) verformt wird und das Auslöseelement (600) die Öffnungsmittel (80) betätigt, so dass bei einer Inhalation durch den Inhalationsansatz (200) ein Behälter durch die Öffnungsmittel geöffnet wird.

7. Vorrichtung nach einem der vorhergehenden Ansprüche, wobei die Öffnungsmittel (80) ein Durchstechelement aufweisen, welches in Bezug auf den Hauptkörper (10) fixiert und dafür geeignet ist, eine Verschlusswand des Behälters derart abzuschneiden, dass der abgeschnittene Teil oder die abgeschnittenen Teile nicht die gebildete(n) Öffnung(en) blockiert (blockieren).

## Claims

1. A fluid inhalation device triggered by inhalation, including a main body (10) and at least one cover element (11, 12) that is mounted to pivot on said main body (10) between a closed position and an open position, said device including at least one individual reservoir containing a single dose of fluid, such as powder, opening means (80) being provided for opening an individual reservoir each time the device is actuated, said device including movable support means (50) that are adapted to move an individual reservoir against said opening means (80) on each actuation, said movable support means (50) being displaceable between a non-dispensing position and a dispensing position, said movable support means (50) being urged towards their dispensing position by resilient means (70), such as a spring, and being held in their non-dispensing position by blocking means that are released by the user inhaling, said device including a cocking member (800) that co-operates with said resilient means (70) and with a cam surface (51) that is formed on said movable support means (50), the fluid dispenser device being **characterized in that** said cam surface (51) comprises at least three cam portions with slopes that are different, having at least one loader portion (511; 511') for loading said resilient means (70), at least one end portion (515; 515') for blocking said cocking member (800) in said open position and/or in said closed position of said at least one cover element (11, 12), and at least one safety portion (513; 513') for interconnecting at least one loader portion (511; 511') with at least one end portion (515; 515'), said cocking member being displaceable over said safety portion (513; 513') without additional compression and without decompression of said resilient means (70), said device being in a stable position when said cocking member (800) is stopped on said safety portion (513; 513').

2. A device according to claim 1, wherein said at least one loader portion (511; 511') is of shape that is plane and rectilinear.

3. A device according to claim 1 or claim 2, wherein said at least one safety portion (513; 513') is in the shape of a circular arc, such that no sideways force is exerted on said cocking member (800) while it is cooperating with said safety portion.

4. A device according to any preceding claim, wherein said at least one end portion (515; 515') is plane or V-shaped.

5. A device according to any preceding claim, wherein, starting from the closed position, said cam surface (51) comprises a loader portion (511), a safety portion (513), and an end portion (515).

6. A device according to any preceding claim, including an inhalation trigger system (60) that comprises a deformable air chamber (61) that co-operates with an inhalation piece (200), and a trigger element (600) that co-operates with said air chamber (61), such that during inhalation through said inhalation piece (200), said air chamber (61) is deformed and said trigger element (600) actuates said opening means (80), such that during inhalation through the inhalation piece (200), a reservoir is opened by said opening means.

7. A device according to any preceding claim, wherein said opening means (80) include a perforator element that is stationary relative to said main body (10) and that is adapted to cut a closure wall of the reservoir in such a manner that the cut portion(s) does/do not obstruct the opening(s) that is/are formed.
